Europäisches Patentamt

European Patent Office

Office européen des brevets

(11) Publication number: **0 397 879**
**A1**

(12) **EUROPEAN PATENT APPLICATION**
**published in accordance with Art.**
**158(3) EPC**

(21) Application number: **89903810.3**

(22) Date of filing: **22.03.89**

(86) International application number:
**PCT/JP89/00300**

(87) International publication number:
**WO 89/11098 (16.11.89 89/27)**

(51) Int. Cl.⁵: **G01N 33/53, G01N 33/543,**
**G01N 33/544**

(30) Priority: **02.05.88 JP 107510/88**

(43) Date of publication of application:
**22.11.90 Bulletin 90/47**

(84) Designated Contracting States:
**DE FR GB IT**

(71) Applicant: **P.C.C. TECHNOLOGY INC.**
**8-7, Shibuya 2-chome**
**Shibuya-ku, Tokyo 150(JP)**

(72) Inventor: **KITANI, Shigekazu Mitsui**
**Petrochemical Ind. Ltd.**
**1-2, Waki 6-chome Waki-cho**
**Kuga-gun Yamaguchi-ken 740(JP)**

(74) Representative: **Myerscough, Philip Boyd et al**
**J.A.Kemp & Co. 14, South Square Gray's Inn**
**London, WC1R 5EU(GB)**

(54) METHOD FOR DETECTING GLYCOSIDICALLY LINKED LOW-MOLECULAR COMPOUND BY IMMUNOASSAY.

(57) A method for detecting and determining a glycosidically linked low-molecular compound, which comprises adsorbing a protein onto a support, adding thereto a specimen containing a glycosidically linked low-molecular compound, treating them with a crosslinking angent capable of crosslinking the protein and the low-molecular compound to indirectly bind the low-molecular compound to the support, and conducting immunoassay.

SPECIFICATION

METHOD FOR DETECTING GLYCOSIDICALLY LINKED LOW-
MOLECULAR COMPOUND BY IMMUNOASSAY

Technical Field of the Invention

The present invention relates to a method for detecting glycosylated low molecular weight compounds by means of immunoassay and to a method for determining the same.

Background of the Invention

Immunoassay which is characterized by the capability of determining with high sensitivity and precision is important technology in the field of drugs and clinical laboratory test. As a conventional method for detecting and determining so called hapten-low molecular weight compounds, the method called "ELISA method" has been generally used. Because of the difficulty in determinative adsorption of low molecular weight compounds on a support, the above ELISA method utilizes the procedure of indirectly detecting and determining low molecular weight compounds in which an antibody for hapten is adsorbed on a support, and then a labelled antigen prepared by labelling the low molecular weight compounds with enzyme is allowed to compete against the low molecular weight compounds in a sample, followed by determining the labelled antigen. The above conventional ELISA method is described in "Immunoassay for Enzyme"

- 1 -

compiled by Kitagawa, Nambara and Ishikawa (Separate volume
No. 31 for Protein Nucleic-acid Enzyme, 1987, edited by
Kyohritsu Shuppan Co., Ltd.).

In the field of immunoassay, nothing has been known or
disclosed regarding the method wherein hapten (glycosylated
low molecular weight compound) is adsorbed on a support
using protein as a medium, while crosslinking agent is used
as is the case with the method of the present invention.
The above fact renders the method of the present invention
novel and unobvious.

In principle, hapten is comparatively low molecular
weight compound which is capable of combining with specific
antibody, but incapable of deriving immune response by
itself (no immunogenicity). Since immunoassay is the
technique which solely utilizes the capability of combining
with the antibody, the combination of hapten with protein
which gives immunogenicity to hapten is entirely
unnecessary. In addition, as an enzyme of delicate nature
is used in Enzyme-immunoassay (EIA), it is easily
anticipated that the delicate enzyme-activity is affected by
the process of combining with protein which is conducted to
provide hapten with immunogenicity. Under such
circumstances, in the state of the art, the combination of
hapten with protein is excluded rather than unexpected.

As described hereinbefore, ELISA method is being used at the present time as the general method of detecting low molecular weight compounds. However, in the case that ELISA method is put into practice, the troublesome procedures such as preparation of labelled antigen by labelling low molecular weight compounds, investigation on setting labelled-antigen concentration and the like are necessary.

The present invention has been made to solve the above disadvantage. In more detail, from the viewpoint that the improvement of the conventional ELISA method cannot lead to a decisive solution to the problems, the present invention has been accomplished for the purpose of realizing the most fundamental technique of allowing hapten to be directly adsorbed onto a support, which has so far been deemed impossible.

Disclosure of the Invention

In order to eliminate the troublesome process of preparing labelled antigen and the like, the inventors intensively studied on the method of conducting immunoassay after allowing glycosylated low molecular weight compounds that are to be determined to determinatively combine with a support. As a result, the inventors have found that hapten (glycosylated low molecular weight compound) is easily and smoothly combine with a support by a protein medium, and further that the use of a crosslinking agent improves the result. Further research and investigation on the basis of the novel knowledge and information finally completed the present invention.

It has been considered to be extremely difficult or even impossible to detect and determine low molecular weight compounds by immunoassay after allowing the compounds to be directly adsorbed on a support such as microtiter plates, beads, membranes, etc. By conversion of conception and intensive research, however, the inventors dared to challenge to the above state of the art, focusing great attention to the combination with protein.

As a result of further research, the inventors have found that the novel procedure in which some protein is adsorbed on the above support, and then a sample containing glycosylated low molecular weight compounds is added together with a crosslinking agent which combines the above compounds and protein to combine the compounds with the protein that has been adsorbed on the above support, followed by immunoassay really enables to detection and determination of the glycosylated low molecular weight compounds. Thus, the present invention has been completed on the basis of the novel information and knowledge as mentioned above.

The most important essence of the method according to the present invention resides in that some protein is adsorbed on a support, sample containing glycosylated low molecular weight compounds to be detected is added to the support, treating with a crosslinking agent capable of crosslinking the compounds with the protein, thereby indirectly combining the compounds with the support, and thereafter the detection and determination of the glycosylated low molecular weight compounds are effected by means of immunoassay.

Brief Description of the Drawing

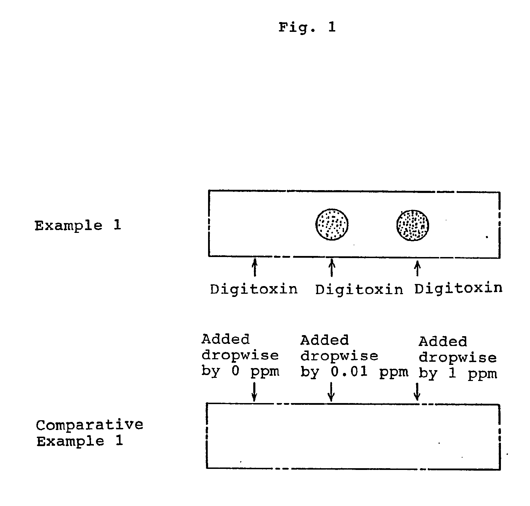

Fig. 1 illustrates the results as obtained in Example 1 and Comparative example 1.

Best Mode of Carrying Out the Invention

The examples of supports of the present invention are, for example, microtiter, plates, beads, membranes, etc., the materials of construction being polyester, polystyrene, polyethylene, polyvinyl chloride, nitrocellulose, nylon, glass, paper, etc. In addition, silicon rubber, polystyrene balls, erythrocyte, inactive albumin particles, etc. can also be used as supports. In summary, a support that can be used in immunoassay can be arbitrarily used as a support of the present invention.

Among the supports as mentioned above, microtiter plates are suitable for mechanizing or automating the immunoassay, since they enable all the operation thereon such as fixation of hapten, sample addition, antigen-antibody reaction, enzymatic reaction, spectroscopic analysis, calorimetry, etc.

Proteins to be adsorbed on such supports are exemplified, in a wide range, by BSA (Bovine serum albumin), cycloglobulin, synthetic polylysine, albumin, erythrocyte, etc. In addition, glycosylated low molecular weight compounds to be fixed on a support are defined as all compounds capable of preparing antibody as hapten.

For the purpose of combining glycosylated low molecular weight compounds with a support by use of protein as a medium, both the method in which glycosylated low molecular weight compounds and protein are bonded together using a crosslinking agent, followed by adsorption onto the support and the method in which a protein is adsorbed on a support, and then the glycosylated low molecular weight compounds are combined with the support by use of a crosslinking agent can be adopted. In this case, the most suitable method free from denaturation of protein or enzyme which is used as a label may be selected in accordance with the types of support, protein, crosslinking agent, and glycosylated low molecular weight compounds.

As a crosslinking agent which combines protein and glycosylated low molecular weight compounds in the method of the present invention, that which is usually used in the same technological field can be arbitrarily used. However, the preferable agent is, for example, metaperiodic acid,

- 6 -

which acts upon the sugar part of the compounds to form dialdehyde, produces schiff base with ε-amino group of the protein, and constitutes the complex of the glycosylated compounds with the protein. As the method of crosslinking glycosylated compounds with protein, the conventional methods as described in "Enzyme-Antibody, revised edition" pp.32 to 34 (Gakusai Kikaku), compiled by Keiichi Watanabe, Kazuko Nakane, and "Lecture on Experimental Biology No. 15, Plant Physiology [1]" pp.75 to 76, compiled by Tadayuki Katsumi, Yoshio Masuda can be utilized as the occasion may demand.

The method according to the present invention enables a wide range of determination for glycosylated low molecular weight compounds, which are exemplified by phenol glycosides such as arctiin, vanillin, etc.; nitrile glycosides such as amygdalin, sambunigrin, etc.; coumarin glycosides such as esculin, kenilin, etc.; anthracene glycosides such as chrysophananein, sennoside, etc.; terpene glycosides such as stevioside, verbenalin, etc.; bitter glycosides such as gentiopicrate, condurangin, etc.; flavone glycosides such as acaciin, apiin, etc.; isoflavone glycosides such as iridin, ononin, etc.; flavonal glycosides such as astragalin, avicularin, etc.; flavanon glycosides such as eriodictin, sakuranin, etc.; cyanidin glycosides such as idein, chrysanthemin, etc.; delphinidin glycosides such as oenin, delphinin, etc.; steroid glycosides such as osladin, digitogenin, etc.; triterpenoid glycosides such as aciatycoid, glycyrrhizin, etc.; cardiac glycosides such as digitoxin, digoxin, etc.; indoxil glycosides; azoxy glycosides; alkaloid glycosides; gibberellin glycosides; lignan glycosides, etc. In addition to the above-mentioned glycosides, N,-glycosides (nitrogen glycosides) are also included in the scope of glycosylated low molecular weight compounds.

In the method of the present invention, the above exemplified glycosides or N-glycosides are directly determined, or can be indirectly determined by the procedure in which the low molecular weight compounds to be finally determined are once glycosylated to form glycosides, which are then determined by the method of the present invention and converted to the object compounds.

As compared with the direct method, the indirect method is particularly useful for improving stability and analytical precision by glycosylating the low molecular weight compounds and shortening analysis hour. Furthermore, even if the object compounds are difficult or impossible to analyze as such, the glycosylation of the compounds enables analysis or improvement or analytic precision, rendering itself particularly useful.

For the purpose of converting object low molecular weight compounds into glycosides, the conventional methods of synthesizing glycosides, for example, Michael method, Fischer method, Königs-Knorr method, Helferich method, Mercaptar method, transglycosylation method and the like may be utilized as the occasion may demand.

In carrying out the method of the present invention, the above-mentioned procedure may be followed, that is, glycosylated low molecular weight compounds in a sample are bonded to a support with crosslinking agent by use of some protein as a medium, then antibody for the compound is prepared, and the treatment operation is made according to the conventional method of immunoassay by utilizing the antibody thus prepared.

The labels to be used for labelled antibody may be those in general use such as enzyme, fluorescent substance, ferritin, radioisotope, etc. The glycosylated low molecular weight compounds may be determined by taking advantage of the characteristics of the label thus used after the reaction with the above compounds. (Change from substrate to product may be measured for enzyme; existence of fluorescence or ferritin for fluorescent substance or ferritin, respectively; radioactivity for radioisotope).

Where the enzyme-labelled-antibody method is utilized in the present invention, the enzymas such as β-galactosidase, peroxidase, glucose oxidase, alkali phosphadase, glucose-6-phosphoric-acid dehydrogenase, etc. are used, and the enzyme-labelled-antibody is prepared by the known method such as glutaraldehyde method, periodic acid method, maleimide method, pyridil-disulfide method and the like. Then, the glycosylated low molecular weight compounds are subjected to antigen-antibody reaction and added by enzyme substrate to form the product. For instance, color-producing (color losing, color-changing) product is determined by way of spectrophotometry, spectrofluorometry, chemical photometry, etc.

The example which follows specifically illustrates the method of the present invention in more detail in comparison with the comparative example:

Example 1

Digitoxin, a type of cardiac glycoside is detected by the following procedure:

(1)   Preparation of antibody

Digitoxin antibody is prepared by the method of Smith, T.W. et al. [J. Pharmacal Exp. Ther., 175, 352(1970)]. Physiological saline solution at a dilution of 1,000 times containing 1% BSA is used for immunoassay.

(2)   Digitoxin detection procedure

①   To a sample solution containing digitoxin are added BSA (Bovine Serum Albumin) and sodium metaperiodate (SMPI) so as to result in concentrations of

10 mg/mℓ of BSA and of 2 mg/mℓ of SMPI.  The resultant mixture is allowed to react at 4°C for 30 minutes.

② The reaction solution of 5 µℓ as prepared in ① is added dropwise to a nitrocellulose membrane, and then dried.

③ The nitrocellulose membrane is immersed in the physiological saline solution containing 10 mg/mℓ BSA and allowed to stand for 30 minutes.

④ The nitrocellulose membrane is taken out and immersed in the digitoxin antibody as described in (1), and allowed to react for 30 minutes.

⑤ The nitrocellulose membrane subjected to reaction with digitoxin antibody is immersed in physiological saline solution, and thoroughly washed.

⑥ The nitrocellulose membrane is immersed in peroxidase-labelled secondary antibody (Dilution of 300 times, made by Wako Pure Chemical Reagent Co., Ltd.) and allowed to react for 30 minutes.

⑦ The nitrocellulose membrane subjected to reaction with the secondary antibody is immersed in physiological saline solution, and thoroughly washed.

⑧ The nitrocellulose membrane is immersed in color-producing liquid containing 3,3'-diaminobenzidin and aqueous hydrogen peroxide, and allowed to react at room temperature for 20 minutes.  The color-producing liquid is prepared according to the description in "Enzyme-Antibody, revised edition" pp.70 (Gakusai Kikaku), compiled by Keiichi Watanabe, Kazuko Nakane.

⑨ The nitrocellulose membrane is washed with water and dried.

⑩ The nirocellulose membrane which produces color by the above operation is shown in Fig. 1.

Comparative example 1

The same procedure as that in Example 1 is applied except that sodium metaperiodate as crosslinking agent is not added. The result is shown in the column of Comparative example in Fig. 1.

Availability in Industry

The immunoassay according to the present invention is the first method which enables the detection and determination of glycosylated low molecular weight compounds acting as hapten without the use of labelled antigen. In addition, different from the competitive determination method in ELISA method in which only a part of immune equilibrium state is measured, the method of the present invention exhibits a marked effect of improving the limit of detection in the concentration of glycosylated low molecular weight compounds to be detected, since the total weight of immunoreaction product is measured.

In particular, most of pharmaceuticals of natural origin consist of glycosides.  Consequently, determination of pharmaceuticals of natural origin is of utmost importance in decision of optimum dose, clinical laboratory test and research on metabolism, and has been made industrially possible for the first time by the present invention.

Furthermore, the low molecular weight compounds which have been difficult or even impossible to determine so far can be determined as the case may be by means of glycosylating the compounds.  That is one of the prominent features of the present invention.

What is claimed is:

1.   A method for detecting a glycosylated low molecular weight compound by means of immunoassay characterized in that a sample containing said glycosylated low molecular weight compound to be detected is added to a protein adsorbate on a support and treated by a crosslinking agent, followed by immunoassay.

2.   The method as set forth in claim 1 characterized in that said crosslinking agent to be used is metaperiodic acid.

Fig. 1

Example 1

↑ Digitoxin  ↑ Digitoxin  ↑ Digitoxin

Added
dropwise
by 0 ppm

Added
dropwise
by 0.01 ppm

Added
dropwise
by 1 ppm

↓ ↓ ↓

Comparative
Example 1

**I. CLASSIFICATION OF SUBJECT MATTER** (if several classification symbols apply, indicate all) 6

According to International Patent Classification (IPC) or to both National Classification and IPC

Int. Cl$^4$   G01N33/53, G01N33/543, G01N33/544

**II. FIELDS SEARCHED**

Minimum Documentation Searched 7

| Classification System | Classification Symbols |
|---|---|
| IPC | G01N33/53, G01N33/543, G01N33/544 |

Documentation Searched other than Minimum Documentation
to the Extent that such Documents are Included in the Fields Searched 8

Jitsuyo Shinan Koho                    1971 - 1989
Kokai Jitsuyo Shinan Koho              1971 - 1989

**III. DOCUMENTS CONSIDERED TO BE RELEVANT** 9

| Category * | Citation of Document, 11 with indication, where appropriate, of the relevant passages 12 | Relevant to Claim No. 13 |
|---|---|---|
| X | JP, A, 56-71023 (Dynasciences Corp.) 13 June 1981 (13. 06. 81) & EP, A, 28132 & AU, A, 6295280, & CA, A, 1146853 | 1-2 |
| A | JP, A, 50-123819 (Teikoku Hormone Mfg. Co., Ltd.) 29 September 1975 (29. 09. 75) (Family: none) | 1-2 |
| A | JP, A, 61-100660 (Morinaga Milk Industry Co., Ltd.) 19 May 1986 (19. 05. 86) (Family: none) | 1-2 |
| X | JP, A, 62-8054 (Kanebo, Ltd.) 16 January 1987 (16. 01. 87) (Family: none) | 1-2 |
| Y | JP, A, 61-205865 (Meidensha Electric Mfg. Co., Ltd.) | |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| May 25, 1989 (25. 05. 89) | June 5, 1989 (05. 06. 89) |

| International Searching Authority | Signature of Authorized Officer |
|---|---|
| Japanese Patent Office | |

Form PCT/ISA/210 (second sheet) (January 1985)

International Application No. PCT/JP89/00300

| | | |
|---|---|---|
| FURTHER INFORMATION CONTINUED FROM THE SECOND SHEET | | |
| Y | JP, A, 61-241665 (Toyobo Co., Ltd.)<br>27 October 1986 (27. 10. 86)<br>(Family: none) | 1-2 |
| Y | JP, A, 57-182652 (Enzyme Technology Inc.)<br>10 November 1982 (10. 11. 82)<br>& FI, A, 813796 | 1-2 |

**V.☐ OBSERVATIONS WHERE CERTAIN CLAIMS WERE FOUND UNSEARCHABLE [1]**

This international search report has not been established in respect of certain claims under Article 17(2) (a) for the following reasons:

1.☐ Claim numbers ............, because they relate to subject matter not required to be searched by this Authority, namely:

2.☐ Claim numbers ............, because they relate to parts of the international application that do not comply with the prescribed requirements to such an extent that no meaningful international search can be carried out, specifically:

3.☐ Claim numbers ............, because they are dependent claims and are not drafted in accordance with the second and third sentences of PCT Rule 6.4(a).

**VI.☐ OBSERVATIONS WHERE UNITY OF INVENTION IS LACKING [2]**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (January 1985)